# EUROPEAN PATENT SPECIFICATION

(11) **EP 2 123 278 B1**
(45) Date of publication and mention of the grant of the patent: **09.01.2013**
(21) Application number: 08704514.2
(22) Date of filing: 07.02.2008
(51) Int. Cl.: A61K 9/00, A61K 31/5575, A61K 47/02, A61K 47/12, A61K 47/18, A61K 47/26

(54) **EYE DROP PREPARATION COMPRISING LATANOPROST**
AUGENTROPFENZUBEREITUNG MIT LATANOPROST
PRÉPARATION DE GOUTTES OPHTALMIQUES COMPRENANT DU LATANOPROST

(30) Priority: 07.02.2007 JP 2007028498; 25.06.2007 JP 2007166788; 21.09.2007 JP 2007245937; 28.12.2007 JP 2007338661
(43) Date of publication of application: 25.11.2009
(73) Proprietor: Teika Pharmaceutical Co., Ltd., Toyama-shi, Toyama 930-0982 (JP)
(72) Inventor: KIMURA, Takahito, Toyama-shi Toyama 930-0982 (JP); WATANABE, Joshu, Toyama-shi Toyama 930-0982 (JP); KOBAYASHI, Minoru, Toyama-shi Toyama 930-0982 (JP); SEKI, Makoto, Toyama-shi Toyama 930-0982 (JP); SHIMATANI, Takao, Toyama-shi Toyama 930-0982 (JP); TAKAGI, Chiharu, Toyama-shi Toyama 930-0982 (JP)
(74) Representative: Blodig, Wolfgang
(86) International application number: PCT/JP2008/052005
(87) International publication number: WO 2008/096804

(56) References cited:
- EP-A1- 1 547 599
- WO-A1-00/03736
- WO-A1-2007/015510
- WO-A2-2006/078659
- JP-A- 2001 515 502
- JP-A- 2002 161 037
- JP-A- 2006 503 913
- US-A1- 2002 103 255

## Description

### Technical Field

This invention relates to an aphthalmic composition for eye drop preparation, more specifically, to an aphthalmic composition for eye drop preparation stably containing latanoprost as an active ingredient.

### Related Art

Glaucoma is a disease that results in visual field constriction caused by damage on the optic nerve due to an increase in intraocular pressure which occurs when aqueous humor produced in the eye is poorly excreted for a certain reason. The intraocular pressure of human is ordinarily within the range of 10 to 21 mmHg, and an intraocular pressure abnormally exceeding the range adversely affects on the optic nerve to cause the constriction of the visual field. Since the visual field that is lost once by glaucoma will never be recovered, glaucoma can be the cause of a visual loss and is the second leading cause of the visual loss in Japan following diabetic retinopathy.

It is possible to treat glaucoma with a drug, laser, or a surgical treatment. In the treatment with laser, outflow of the aqueous humor is promoted by forming a hole in the iris by the laser irradiation or by irradiating the trabecular meshwork with the laser. In the surgical treatment, a method of facilitating a flow of the aqueous humor by forming a pathway by incising a part preventing the flow of aqueous humor, a method of suppressing production of the aqueous humor in the ciliary body, and the like are generally employed.

As the drug treatment, ocular hypotensive action of prostaglandin has recently been noted, and a derivative thereof is used for an eye drop preparation for treating glaucoma or ocular hypertension (Patent Document 1). As a prostaglandin F2α derivative usable as the therapeutic agent for glaucoma, latanoprost (chemical name: isopropyl-(Z)-7[(1R,2R,3R,5S)3,5-dihydroxy-2-[(3R)-3-hydroxy-5-phenylpentyl]cyclopentyl]-5-heptanoate) has been known (Patent Document 2), and an eye drop preparation (trade name: Xalatan ophthalmic solution) using latanoprost as an active ingredient is marketed by Pfizer Japan Inc.

The ocular hypotensive action of latanoprost is considered to be achieved by the promotion of outflow from uveoscleral pathway among pathways for the aqueous humor, and the ophthalmic solution marketed as Xalatan (trade name) contains 0.005 (w/v)% of latanoprost as an active ingredient.

However, since there are problems that latanoprost is easily degraded in water and tends to adsorb onto an inner surface of a container when latanoprost is stored particularly in a plastic container, storage of the above-mentioned commercially available product at a room temperature is not admitted, and the product is required to be stored at a cool and dark place, thereby entailing a drawback of poor usability.

Particularly, though it is assured by a stability test that a certain level of active ingredient content is maintained for 3 years which is the expiry date for use when the eye drop preparation is stored under the above-specified conditions, an active ingredient content after the eye drop preparation is prescribed for a patient to be under the management of the patient is not assured. Specifically, the therapeutic agent for glaucoma or ocular hypertension is often prescribed for an aged person and has a tendency of generally low compliance as compared to other eye drop preparations. Further, in view of possibility that the eye drop preparation is carried during travel, it is considered that the low temperature storage is not actually practiced in many cases despite instruction of the low temperature storage from a doctor or pharmacist to the patient.

A volume of the latanoprost eye drop preparation (trade name: Xalatan ophthalmic solution) is set to that which is used up in about 4 weeks under ordinary use conditions (about 2.5 ml/bottle), but, since there is a possibility that active ingredient content decreases during the period in which the preparation is not stored at a low temperature and its effect becomes insufficient at a later stage of the use period, there has been a demand for an eye drop preparation that can be stored at an ordinary temperature.

Various proposals have been made in order to solve this problem, and, for example, Patent Document 3 reports that it is possible to provide a stable aqueous drug composition in which degradation of the prostaglandin F2α derivative is suppressed by forming the prostaglandin F2α derivative containing latanoprost into an oil-in-water emulsion with the use of oil, a water soluble polymer, and water.

Also, Patent Document 4 reports that it is possible to provide an ophthalmic solution wherein latanoprost is so stabilized as to be stored at a room temperature by at least one means selected from (1) a means of adjusting pH to 5.0 to 6.25 and (2)a means of adding ε-aminocaproic acid.

Further, Patent Document 5 discloses a method for suppressing degradation of latanoprost in an ophthalmic solution containing latanoprost by adding trometamol to the ophthalmic solution.

Patent Document 6 and its divisional application Patent Document 7 report that it is possible to largely enhance chemical stability of a prostaglandin composition having a structure similar to that of latanoprost by using polyethoxized castor oil. However, these publications do not give data and suggestion sufficient for alleging that latanoprost is stabilized.

Also, Patent Document 8 discloses that an aqueous prostaglandin formulation is stable in a polypropylene container. However, this publication does not give data and suggestion sufficient for alleging that latanoprost is stabilized.

As described above, various studies have been conducted in the related art on the method for improving the stability of the latanoprost ophthalmic solution, but, investigation on adsorption of latanoprost is insufficient since the experiments disclosed in these publications are conducted by using a glass ampoule, not a plastic container, or it has not been clarified whether or not satisfactory stability is achieved in the case where the ophthalmic solution containing latanoprost is stored in a plastic container since examination is conducted by using prostaglandins having chemical structures that are different from latanoprost. Thus, as a present status, solution to the problem of obtaining a stable latanoprost eye drop preparation has not been found yet.

Patent Document 1: Japanese Patent No. 3612178
Patent Document 2: Japanese Patent No. 2721414
Patent Document 3: WO2005/044276
Patent Document 4: JP-A-2004-182719
Patent Document 5: JP-A-2007-63265
Patent Document 6: JP-T-11-500122
Patent Document 7: JP-A-2005-015498
Patent Document 8: JP-T-2002-520368
Prostaglandin-containing composition are known from WO 2006/078659.

### Disclosure of the Invention

### Problems to be Solved by the Invention

An object of this invention is to provide an ophthalmic solution capable of preventing not only degradation of latanoprost in water but also adsorption of latanoprost onto a plastic container so that a decrease in latanoprost content thereof is satisfactorily prevented.

### Means for Solving the Problems

The inventors conducted an extensive research in the aim of solving the above-described problems to find that it is possible to suppress degradation of latanoprost in water and to prevent adsorption of latanoprost onto a plastic container surface by adding a specific component to latanoprost, thereby accomplishing this invention.

An eye drop preparation comprises an ophthalmic solution composition comprising components (A) and (B) and packed in a plastic container:
(A) latanoprost; and
(B) a nonionic surfactant.

further, this invention provides an ophtalmic composition for eye drop preparation as defined by claim 1.

Further, this invention provides a composition for any one of the above-described eye drop preparations, which has an appearance of colorless and clear and is contained in an amount of 2.5 mL in a polypropylene or polyethylene container having a diameter of about 1.5 cm and a capacity of about 5 mL, wherein a latanoprost remaining ratio in an ophthalmic solution in any one of the containers after storage for 30 days under the conditions of 40°C, a relative humidity of 75%, shading, and upright still standing is 97.0% or more.

Specifically, this invention provides an ophthalmic composition for eye drop preparation, comprising 0.005 (w/v)% of latanoprost, 0.4 to 1.2 (w/v)% of trometamol, 0.05 to 0.15 (w/v)% of citric acid hydrate, 0.5 to 1.5 (w/v)% of D-mannitol, 0.55 to 1.65 (w/v)% of glycerin, 0.15 to 0.45 (w/v)% of hypromellose, 0. 025 to 0. 375 (w/v)% of Polysorbate 80, and 0.0055 to 0.030 (w/v)% of benzalkonium chloride.

### Effect of the Invention

The eye drop preparation of this invention is obtainable by packing an ophthalmic solution composition containing latanoprost in a plastic container and is prevented from both of degradation of latanoprost in water and adsorption of latanoprost onto an inner surface of the plastic container. Therefore, this invention enables provision of an ophthalmologic agent that is satisfactorily suppressed in decrease in latanoprost content.

Specifically, even an aged person or the like has less difficulty in storing and using the ophthalmic solution of this invention since it is possible to store the ophthalmic solution at an ordinary temperature; the ophthalmic solution has improved convenience since the ophthalmic solution is suitably taken along during travel; and the ophthalmic solution achieves stable ocular hypotensive action since a satisfactory active ingredient content can be maintained during a duration of use.

### Best Mode for Carrying out the Invention

Latanoprost (component (A)) that is used as the active ingredient of the ophthalmic solution composition of this invention is the prostaglandin F2α derivative represented by the chemical name isopropyl-(Z)-7[(1R,2R,3R,5S)3,5-dihydroxy-2-[(3R)-3-hydroxy-5-phenylpentyl)cyclopentyl]-5-heptanoate) as described above. Latanoprost is a selective FP receptor agonist and used as a therapeutic drug for glaucoma since latanoprost has strong ocular hypotensive action due to promotion of outflow of aqueous humor.

Also, a nonionic surfactant is contained as a component (B) in the ophthalmic solution composition of this invention, which is polyoxyethylene sorbitan monooleate (Polysorbate 80).

The organic acid having two or more carboxyl groups to be used as the component (C) is citric acid hydrate.

It is possible to produce the eye drop preparation of this invention by any one of the following methods. The ophthalmic solution composition prepared by using the components (A) to (C) and organic amine as required and mixing them by a known general process is packed in a plastic container. Preferred examples of the preparation method of the ophthalmic solution composition include a method of mixing purified water, latanoprost (component (A)), and the nonionic surfactant (the component (B)) and dissolving with heating when so required, followed by addition of other components.

In the preparation of the ophthalmic solution composition of this invention, additives such as a buffering agent, a tonicity agent (buffering agent and tonicity agent are those other than the essential components of this invention), an antiseptic, a viscosity improver, a pH adjuster, an algefacient may be added in addition to the above-described components when so required.

As the buffering agent among the additives, a known buffering agent that is ordinarily used for ophthalmic solutions may be used without limitation. Examples of the known buffering agent include aminoethyl sulfonic acid; epsilon-aminocaproic acid; a citric acid buffering agent such as citric acid and sodium citrate; an acetic acid buffering agent such as acetic acid, potassium acetate, and sodium acetate; a carbonate buffering agent such as sodium hydrogen carbonate and sodium carbonate; a boric acid buffering agent such as boric acid and borax; a phosphoric acid buffering agent such as disodium hydrogenphosphate, sodium dihydrogenphosphate, dipotassium hydrogenphosphate, potassium dihydrogenphosphate, and hydrates thereof; and the like. The buffering agent may be used alone or in combination of two or more kinds.

A content of the buffering agent in the ophthalmic solution of this invention may ordinarily be 0.01 to 5.0 (w/v)%, preferably 0.05 to 2.0 (w/v)%, more preferably 0.1 to 1.0 (w/v)%, though the content is varied depending on the type of the buffering agent and cannot be defined flatly. The eye irritation is suppressed within the above range.

As the tonicity agent, a known tonicity agent that is ordinarily used for ophthalmic solutions may be used. Examples of the tonicity agent include a water soluble polyhydric alcohol such as glycerin and propylene glycol; inorganic salt such as sodium chloride and potassium chloride; and the like. The tonicity agent may be used alone or in combination of two or more kinds.

A content of the tonicity agent in the ophthalmic solution of this invention may ordinarily be 0.01 to 5.0 (w/v)%, preferably 0.02 to 3.0 (w/v)%, more preferably 0.05 to 2.0 (w/v)%, though the content is varied depending on the type of the tonicity agent and cannot be defined flatly. The eye irritation is suppressed within the above range.

As the antiseptic, a known antiseptic that is ordinarily used for ophthalmic solutions may be used without limitation within the range that does not impair the stability of the active ingredients and within the acceptable range in terms of formulation. Examples of the known antiseptic include sorbic acid, potassium sorbate, p-hydroxybenzoate ester (p-hydroxybenzoate methyl, p-hydroxybenzoate ethyl, p-hydroxybenzoate propyl, p-hydroxybenzoate butyl, etc.), chlorhexidine gluconate, quaternary ammonium salt (benzalkonium chloride, benzetonium chloride, cetylpyridinium chloride, etc.), alkylpolyaminoethyl glycine, chlorobutanol, polyquad, polyhexamethylene biguanide, chlorhexidine, and the like.

Further, examples of the viscosity improver include a cellulose-based viscosity improver such as dextran, hydroxypropyl methylcellulose (hypromellose), hydroxyethylcellulose, hydroxypropylcellulose, carboxymethylcellulose, and methylcellulose, a carboxyvinyl polymer, polyvinyl alcohol, gum arabic, alginic acid, povidone, a xanthan gum, and the like. A content in the case of adding the cellulose-based viscosity improver may ordinarily be 0.01 to 1.0 (w/v)%, preferably 0.05 to 0.7 (w/v)%, more preferably 0.1 to 0.4 (w/v)%, though the content is varied depending on the type of the viscosity improver and cannot be defined flatly. Within the above range, the eye drop preparation is comfortable to apply and suppressed in eye irritation. The cellulose-based viscosity improver may be used alone or in combination of two or more kinds. Among the above, hypromellose is used from the viewpoint of latanoprost stability.

Examples of the pH adjuster include hydrochloric acid, phosphoric acid, sodium hydroxide, potassium hydroxide, sodium carbonate, sodium hydrogen carbonate, and the like, and examples of the algefacient include menthol, camphol, borneol, geraniol, eucalyptus oil, mint oil, and the like.

Also, it is possible to further add an active ingredient such as a decongestant, an eye muscle accommodator, an antiphlogistic/styptic, a vitamin, amino acid, an antibacterial agent, and the like as required insofar as the active ingredient does not cause a problem such as eye irritation.

As the decongestant, a known inorganic salt that is ordinarily used for ophthalmic solutions may be used without limitation within the range that does not impair the stability of active ingredients and within the acceptable range in terms of formulation. Examples of the known decongestant include epinephrine, epinephrine hydrochloride, ephedrine hydrochloride, tetrahydrozoline hydrochloride, naphazoline hydrochloride, naphazoline nitrate, phenylephrine hydrochloride, methyl ephedrine hydrochloride, and the like.

As the eye muscle accommodator, a known eye muscle accommodator that is ordinarily used for ophthalmic solutions may be used without limitation within the range that does not impair the stability of active ingredients and within the acceptable range in terms of formulation. Examples of the known eye muscle accommodator include neostigmine methylsulfate and the like.

As the antiphlogistic/styptic, a known antiphlogistic/styptic that is ordinarily used for ophthalmic solutions may be used without limitation within the range that does not impair the stability of active ingredients and within the acceptable range in terms of formulation. Examples of the known antiphlogistic/styptic include epsilon-aminocaproic acid, allantoin, berberine chloride, berberine sulfate, sodium azulene sulfonate, dipotassium glycyrrhizinate, zinc sulfate, zinc lactate, lysozyme chloride, and the like.

As the vitamin, a known vitamin that is ordinarily used for ophthalmic solutions may be used without limitation within the range that does not impair the stability of active ingredients and within the acceptable range in terms of formulation. Examples of the known vitamin include flavin adenine dinucleotide sodium, cyanocobalamin, retinol acetate, retinol palmitate, pyridoxine hydrochloride, panthenol, calcium pantothenate, sodium pantothenate, tocopherol acetate, and the like.

As the amino acid, a known amino acid that is ordinarily used for ophthalmic solutions may be used without limitation within the range that does not impair the stability of active ingredients and within the acceptable range in terms of formulation. Examples of the known amino acid include potassium L-asparaginate, magnesium L-asparaginate, magnesium/potassium L-asparaginate (equal parts mixture), aminoethyl sulfonic acid, sodium chondroitin sulfate, and the like.

As the antibacterial agent, a known antibacterial agent that is ordinarily used for ophthalmic solutions may be used without limitation within the range that does not impair the stability of active ingredients and within the acceptable range in terms of formulation. Examples of the known antiseptic include sulfamethoxazole, sulfamethoxazole sodium, sulfisoxazole, sulfisomidine sodium, ofloxacin, norfloxacin, and the like.

A pH level of the ophthalmic solution composition according to this invention may ordinarily be 5 to 9, preferably 6 to 8, more preferably 6.5 to 7.5. When the pH level deviates from the range of 5 to 9, irritation can be felt in ocular instillation, and the latanoprost stability is undesirably deteriorated.

An osmotic pressure of the ophthalmic solution composition of this invention may ordinarily be adjusted to 100 to 400 mOsm, preferably 200 to 350 mOsm, particularly preferably 250 to 300 mOsm. The osmotic pressure within the above-specified ranges can suppress irritation in ocular instillation. It is possible to keep the above-specified osmotic pressure range by containing the tonicity agent in the above-described range.

An osmotic pressure ratio of the ophthalmic solution composition according to this invention may ordinarily be 0.5 to 1.5, preferably 0.7 to 1.3, more preferably 0.9 to 1.1. When the osmotic pressure ratio deviates from the range of 0.5 to 1.5, irritation can undesirably be felt in ocular instillation.

Since the eye drop preparation composition of this invention is suppressed in latanoprost adsorption onto the plastic container, it is possible to use general plastic containers for eye drop preparations for packing the eye drop preparation composition. For example, as a material for the container, it is possible to use a thermoplastic resin such as polyethylene, polypropylene, polyethylene terephthalate, polyethylene naphthalate, polyallylate, and a polycarbonate ethylene/vinyl alcohol copolymer insofar as the thermoplastic resin is satisfactory in terms of cost, strength, optical transmittance, gas or water vapor barrier property (moisture permeation), and the like. Examples of a preferred polymer alloy include a polymer blend of a plurality of synthetic resins (polymer blend of polyethylene terephthalate and polyethylene naphthalate, etc.). Also, the plastic container may preferably have a transparency that allows the contained solution and insoluble contaminants to be seen from the outside. Among others, from the viewpoints of a balance between moisture permeation and adsorption of active ingredients, polyethylene, polypropylene, and polyethylene terephthalate are preferred, among which polyethylene and polypropylene are more preferred due to its property of great flexibility, and polyethylene is particularly preferred.

A shape of the container is not particularly limited insofar as the container can contain a liquid, but the container generally has a cylindrical shape. It is possible to appropriately select a capacity of the container in the case of a multi-dose container, for example, from a range of about 0.5 to 20 mL, and the capacity may preferably be 1 to 10 mL, more preferably 2 to 5 mL. Further, the eye drop preparation container may be covered with a shrinkable film as required after packing the eye drop preparation composition of this invention.

It is possible to select dosage and administration of the ophthalmic solution of this invention depending on symptom of a glaucoma patient requiring instillation, the active ingredients contained in the ophthalmic solution, and formulation. For example, in the case where only latanoprost is contained as the active ingredient, instillation is once per day, in general, and about one drop is applied per instillation.

An ophthalmic composition for eye drop preparation comprising 0.005 (w/v)% of latanoprost, 0.4 to 1.2 (w/v)% of trometamol, 0.05 to 0.15 (w/v)% of citric acid hydrate, 0.5 to 1.5 (w/v)% of D-mannitol, 0.55 to 1.65 (w/v)% of glycerin, 0.15 to 0.45 (w/v)% of hypromellose, 0.025 to 0.375 (w/v)% of polysorbate 80, and 0.0055 to 0. 030 (w/v)% of benzalkonium chloride is preferred.

The ophthalmic solution of this invention described above is packed in the polypropylene or polyethylene container (not covered with the shrinkable film), and the container is stopped up with an inner spigot normally fitted with the container and a cap to give an eye drop preparation, and the eye drop preparation achieves a latanoprost remaining ratio in the ophthalmic solution after 30 days of storage under the conditions of 40°C, relative humidity of 75%, shading, and upright still standing of 97% or more, preferably 98% or more, more preferably 99% or more. Therefore, the eye drop preparation is capable of maintaining the satisfactory active ingredient content during a duration of use when stored at an ordinary temperature.

### Examples

Contents of this invention will be described in detail in the following Examples and Test Examples, but this invention is not limited to the contents.

### Example 1 (Reference)

0.005 g of latanoprost and 0.25 g of polysorbate 80 that had been mixed previously were added to and dissolved into a small amount of purified water that had been heated to about 80°C. After returning the solution to a room temperature, 0.8 g of trometamol and 0.1 g of citric acid hydrate were added and dissolved into the solution, and a pH level was adjusted to about 6.7 by using diluted hydrochloric acid or sodium hydroxide. 0.54 g of sodium chloride was added to and dissolved into the solution, and purified water was added to adjust a total amount to 100 mL, thereby obtaining an ophthalmic solution composition. The ophthalmic solution composition had a pH level of 6.72 and an osmotic pressure ratio of 1.01.

### Example 2 (Reference)

0.005 g of latanoprost and 0.25 g of polysorbate 80 that had been mixed previously were added to and dissolved into a small amount of purified water that had been heated to about 80°C. After returning the solution to a room temperature, 0.8 g of monoethanolamine and 0.1 g of citric acid hydrate were added and dissolved into the solution, and a pH level was adjusted to about 6.7 by using diluted hydrochloric acid or sodium hydroxide. 0.18 g of sodium chloride was added to and dissolved into the solution, and purified water was added to adjust a total amount to 100 mL, thereby obtaining an ophthalmic solution composition. The ophthalmic solution composition had a pH level of 6. 65 and an osmotic pressure ratio of 1.01.

### Example 3 (Reference)

0.005 g of latanoprost and 0.25 g of polyoxyethylene hydrogenated castor oil that had been mixed previously were added to and dissolved into a small amount of purified water that had been heated to about 80°C. After returning the solution to a room temperature, 0.8 g of trometamol and 0.1 g of citric acid hydrate were added and dissolved into the solution, and a pH level was adjusted to about 6.7 by using diluted hydrochloric acid or sodium hydroxide. 0.55 g of sodium chloride was added to and dissolved into the solution, and purified water was added to adjust a total amount to 100 mL, thereby obtaining an ophthalmic solution composition. The ophthalmic solution composition had a pH level of 6.86 and an osmotic pressure ratio of 1.00.

### Example 4 (Reference)

0.005 g of latanoprost and 0.25 g of polysorbate 80 that had been mixed previously were added to and dissolved into a small amount of purified water that had been heated to about 80°C. After returning the solution to a room temperature, 0.1 g of citric acid hydrate was added and dissolved into the solution, and a pH level was adjusted to about 6.7 by using diluted hydrochloric acid or sodium hydroxide. 0.84 g of sodium chloride was added to and dissolved into the solution, and purified water was added to adjust a total amount to 100 mL, thereby obtaining an ophthalmic solution composition. The ophthalmic solution composition had a pH level of 6.63 and an osmotic pressure ratio of 1.00.

### Comparative Example 1

0.005 g of latanoprost and 0.25 g of polysorbate 80 that had been mixed previously were added to and dissolved into a small amount of purified water that had been heated to about 80°C. After returning the solution to a room temperature, 0.8 g of trometamol was added and dissolved into the solution, and a pH level was adjusted to about 6. 7 by using diluted hydrochloric acid or sodium hydroxide. 0.51 g of sodium chloride was added to and dissolved into the solution, and purified water was added to adjust a total amount to 100 mL, thereby obtaining an ophthalmic solution composition. The ophthalmic solution composition had a pH level of 6. 68 and an osmotic pressure ratio of 1.01.

### Comparative Example 2

0. 005 g of latanoprost was added to and dissolved into a small amount of purified water that had been heated to about 80°C. After returning the solution to a room temperature, 0.8 g of trometamol and 0.1 g of citric acid hydrate were added and dissolved into the solution, and a pH level was adjusted to about 6.7 by using diluted hydrochloric acid or sodium hydroxide. 0.55 g of sodium chloride was added to and dissolved into the solution, and purified water was added to adjust a total amount to 100 mL, thereby obtaining an ophthalmic solution composition. The ophthalmic solution composition had a pH level of 6.85 and an osmotic pressure ratio of 0.99.

### Comparative Example 3

0.005 g of latanoprost was added to and dissolved into a small amount of purified water that had been heated to about 80°C. After returning the solution to a room temperature, 0.8 g of trometamol was added and dissolved into the solution, and a pH level was adjusted to about 6. 7 by using diluted hydrochloric acid or sodium hydroxide. 0. 52 g of sodium chloride was added to and dissolved into the solution, and purified water was added to adjust a total amount to 100 mL, thereby obtaining an ophthalmic solution composition. The ophthalmic solution composition had a pH level of 6. 68 and an osmotic pressure ratio of 1.01.

### Comparative Example 4

0.005 g of latanoprost and 0.25 g of polysorbate 80 that had been mixed previously were added to and dissolved into 1 part of purified water that had been heated to about 80°C. After returning the solution to a room temperature, a pH level was adjusted to about 6.7 by using diluted hydrochloric acid or sodium hydroxide. 0.90 g of sodium chloride was added to and dissolved into the solution, and purified water was added to adjust a total amount to 100 mL, thereby obtaining an ophthalmic solution composition. The ophthalmic solution composition had a pH level of 5.55 and an osmotic pressure ratio of 1.01.

### Comparative Example 5

0.005 g of latanoprost was added to and dissolved into a small amount of purified water that had been heated to about 80°C. After returning the solution to a room temperature, 0.1 g of citric acid hydrate was added and dissolved into the solution, and a pH level was adjusted to about 6.7 by using diluted hydrochloric acid or sodium hydroxide. 0.85 g of sodium chloride was added to and dissolved into the solution, and purified water was added to adjust a total amount to 100 mL, thereby obtaining an ophthalmic solution composition. The ophthalmic solution composition had a pH level of 6.71 and an osmotic pressure ratio of 1.00.

### Comparative Example 6

0.005 g of latanoprost and 0.25 g of polysorbate 80 that had been mixed previously were added to and dissolved into a small amount of purified water that had been heated to about 80°C. After returning the solution to a room temperature, 0.8 g of trometamol and 0.8 g of acetic acid were added and dissolved into the solution, and a pH level was adjusted to about 6. 7 by using diluted hydrochloric acid or sodium hydroxide. 0.51 g of sodium chloride was added to and dissolved into the solution, and purified water was added to adjust a total amount to 100 mL, thereby obtaining an ophthalmic solution composition. The ophthalmic solution composition had a pH level of 6.76 and an osmotic pressure ratio of 1.01.

### Test Example (Reference)

### Storage Test

Each of the ophthalmic solution compositions obtained by Reference Examples 1 to 4 and Comparative Examples 1 to 6 and a commercially available product (each 2.5 mL) was packed in glass ampoules, and the ampoules were sealed by welding, followed by storage for 4 days and 8 days at 80°C. A latanoprost content after termination of each of the storage periods was measured by using high speed liquid chromatography. From a difference between each of the latanoprost contents after the storage and a content at the start of the test, a latanoprost remaining ratio was calculated. Results are shown in Table 1. It is considered that the glass ampoule substantially does not absorb latanoprost on its surface.

**[Table 1]**

| | Latanoprost, Remaining ratio (%) | | |
|---|---|---|---|
| | Start of test | After 4 days of storage | After 8 days of storage |
| Example 1Reference | 100.0 | 98.2 | 95.7 |
| Example 2 Reference | 100.0 | 98.1 | 96.9 |
| Example 3 Reference | 100.0 | 98.4 | 97.2 |
| Example 4 Reference | 100.0 | 96.2 | 94.6 |
| Comp. Ex. 1 | 100.0 | 95.0 | 66.4 |
| Comp. Ex. 2 | 100.0 | 93.9 | 92.9 |
| Comp. Ex. 3 | 100.0 | 94.0 | 91.8 |
| Comp. Ex. 4 | 100.0 | 86.9 | 26.6 |
| Comp. Ex. 5 | 100.0 | 88.1 | 82.0 |
| Comp. Ex. 6 | 100.0 | 92.2 | 54.7 |
| Commercially Available Product * | 100.0 | 84.5 | 76.6 |

| | | | |
|---|---|---|---|
| * Xalatan ophthalmic solution (Lot No. 07AH007) | | | |

### Test Example 2 (Reference)

### Adsorption Ratio Measurement Test

Adsorption of latanoprost contained in each of the ophthalmic solution compositions obtained by Reference Examples 1 to 4 and Comparative Examples 1 to 6 and a commercially available product was tested. After filling each of a polyethylene container and a glass ampoule with 2.5 mL of each of the samples, the polyethylene container was provided with an inner cap and an outer cap made from plastic, and the glass ampoule was sealed by welding, thereby giving eye drop preparations.

The eye drop preparations were stored for 7 days under the conditions of upright still standing and 60°C, and a latanoprost content at termination of the storage period was measured by using high speed liquid chromatography. Results are shown in Table 2. The content is expressed by way of a ratio (remaining ratio) to the content at the start of the test. The data of the polyethylene container was corrected by using a moisture evaporation value of a control wherein only purified water was stored under the same conditions.

**[Table 2]**

| | Latanoprost Remaining ratio (%) | | | Latanoprost Adsorption Ratio (%) |
|---|---|---|---|---|
| | Start of test | (1) After 7 days of storage in glass container | (2) After 7 days of storage in polyethylene container | [(1)-(2)] |
| Example 1 Reference | 100.0 | 98.9 | 97.6 | 1.3 |
| Example 2 Reference | 100.0 | 98.9 | 97.8 | 1.1 |
| Example 3 Reference | 100.0 | 98.7 | 97.0 | 1.7 |
| Example 4 Reference | 100.0 | 98.9 | 97.0 | 1.9 |
| Comp. Ex. 1 | 100.0 | 98.2 | 96.9 | 1.3 |
| Comp. Ex. 2 | 100.0 | 94.4 | 79.6 | 14.8 |
| Comp. Ex. 3 | 100.0 | 95.2 | 81.7 | 13.5 |
| Comp. Ex. 4 | 100.0 | 96.3 | 97.0 | -0.7 |
| Comp. Ex. 5 | 100.0 | 91.4 | 80.1 | 11.3 |
| Comp. Ex. 6 | 100.0 | 97.7 | 97.2 | 0.5 |
| Commercially Available Product * | 100.0 | 91.2 | 79.3 | 11.9 |

| | | | | |
|---|---|---|---|---|
| * Xalatan ophthalmic solution (Lot No. 07AH007) | | | | |

From the above results, it was revealed that the formulations of Reference Examples 1 to 4 are excellent in both of adsorption suppression and degradation suppression since the formulations of Reference Examples 1 to 4 have higher latanoprost remaining ratio itself as compared to the formulations of Comparative Examples 1 to 6 and the commercially available product and since the latanoprost adsorption ratio in the plastic container is not largely different from that of the glass container.

From the above results, it was revealed that a problematic reduction in content of the active ingredient does not occur in the composition for latanoprost-containing eye drop preparation and that stable storage is enabled in a state of being packed in a plastic container.

### Example 5 (Reference)

0.005 g of latanoprost and 0.25 g of polysorbate 80 that had been mixed previously were added to and dissolved into a small amount of purified water that had been heated to about 80°C. After returning the solution to a room temperature, 0.8 g of trometamol and 0.1 g of citric acid hydrate were added and dissolved into the solution, and a pH level was adjusted to about 6.7 by using diluted hydrochloric acid or sodium hydroxide. 0.54 g of sodium chloride was added to and dissolved into the solution, and purified water was added to adjust a total amount to 100 mL, thereby obtaining an ophthalmic solution composition. The ophthalmic solution composition had a pH level of 6.72 and an osmotic pressure ratio of 1.01.

### Example 6 (Reference)

0.005 g of latanoprost and 0.25 g of polysorbate 80 that had been mixed previously were added to and dissolved into a small amount of purified water that had been heated to about 80°C. After returning the solution to a room temperature, 0.8 g of monoethanolamine and 0.1 g of citric acid hydrate were added and dissolved into the solution, and a pH level was adjusted to about 6.7 by using diluted hydrochloric acid or sodium hydroxide. 0.18 g of sodium chloride was added to and dissolved into the solution, and purified water was added to adjust a total amount to 100 mL, thereby obtaining an ophthalmic solution composition. The ophthalmic solution composition had a pH level of 6.65 and an osmotic pressure ratio of 1.01.

### Example 7 (Reference)

0.005 g of latanoprost and 0.25 g of polyoxyethylene (60) hardened castor oil that had been mixed previously were added to and dissolved into a small amount of purified water that had been heated to about 80°C. After returning the solution to a room temperature, 0.8 g of trometamol and 0.1 g of citric acid hydrate were added and dissolved into the solution, and a pH level was adjusted to about 6.7 by using diluted hydrochloric acid or sodium hydroxide. 0.55 g of sodium chloride was added to and dissolved into the solution, and purified water was added to adjust a total amount to 100 mL, thereby obtaining an ophthalmic solution composition. The ophthalmic solution composition had a pH level of 6.86 and an osmotic pressure ratio of 1.00.

### Example 8 (Reference)

0.005 g of Latanoprost and 0.25 g of polysorbate 80 that had been mixed previously were added to and dissolved into a small amount of purified water that had been heated to about 80°C. After returning the solution to a room temperature, 0.2 g of glucose and 0.1 g of citric acid hydrate were added and dissolved into the solution, and a pH level was adjusted to about 6.7 by using diluted hydrochloric acid or sodium hydroxide. 0.8 g of sodium chloride was added to and dissolved into the solution, and purified water was added to adjust a total amount to 100 mL, thereby obtaining an ophthalmic solution composition. The ophthalmic solution composition had a pH level of 6.68 and an osmotic pressure ratio of 0.98.

### Example 9 (Reference)

0.005 g of latanoprost and 0.25 g of polysorbate 80 that had been mixed previously were added to and dissolved into a small amount of purified water that had been heated to about 80°C. After returning the solution to a room temperature, 4.0 g of mannitol and 0.1 g of citric acid hydrate were added and dissolved into the solution, and a pH level was adjusted to about 6.7 by using diluted hydrochloric acid or sodium hydroxide. 0.1 g of sodium chloride was added to and dissolved into the solution, and purified water was added to adjust a total amount to 100 mL, thereby obtaining an ophthalmic solution composition. The ophthalmic solution composition had a pH level of 6.72 and an osmotic pressure ratio of 0.97.

### Example 10 (Reference)

0.005 g of latanoprost and 0.25 g of polysorbate 80 that had been mixed previously were added to and dissolved into a small amount of purified water that had been heated to about 80°C. After returning the solution to a room temperature, 0.8 g of sorbitol and 0.1 g of citric acid hydrate were added and dissolved into the solution, and a pH level was adjusted to about 6.7 by using diluted hydrochloric acid or sodium hydroxide. 0.65 g of sodium chloride was added to and dissolved into the solution, and purified water was added to adjust a total amount to 100 mL, thereby obtaining an ophthalmic solution composition. The ophthalmic solution composition had a pH level of 6.69 and an osmotic pressure ratio of 0.93.

### Example 11 (Reference)

0.005 g of latanoprost and 0.25 g of polysorbate 80 that had been mixed previously were added to and dissolved into a small amount of purified water that had been heated to about 80°C. After returning the solution to a room temperature, 0.1 g of dextran 70 and 0.1 g of citric acid hydrate were added and dissolved into the solution, and a pH level was adjusted to about 6.7 by using diluted hydrochloric acid or sodium hydroxide. 0.84 g of sodium chloride was added to and dissolved into the solution, and purified water was added to adjust a total amount to 100 mL, thereby obtaining an ophthalmic solution composition. The ophthalmic solution composition had a pH level of 6.70 and an osmotic pressure ratio of 0.97.

### Example 12 (Reference)

0.005 g of latanoprost and 0.25 g of polysorbate 80 that had been mixed previously were added to and dissolved into a small amount of purified water that had been heated to about 80°C. After returning the solution to a room temperature, 0.1 g of citric acid hydrate was added and dissolved into the solution, and a pH level was adjusted to about 6.7 by using diluted hydrochloric acid or sodium hydroxide. 0.84 g of sodium chloride was added to and dissolved into the solution, and purified water was added to adjust a total amount to 100 mL, thereby obtaining an ophthalmic solution composition. The ophthalmic solution composition had a pH level of 6.63 and an osmotic pressure ratio of 1.00.

### Example 13 (Reference)

0.005 g of latanoprost and 0.25 g of polysorbate 80 that had been mixed previously were added to and dissolved into a small amount of purified water that had been heated to about 80°C. After returning the solution to a room temperature, 0.1 g of sodium edetate hydrate was added and dissolved into the solution, and a pH level was adjusted to about 6.7 by using diluted hydrochloric acid or sodium hydroxide. 0.9 g of sodium chloride was added to and dissolved into the solution, and purified water was added to adjust a total amount to 100 mL, thereby obtaining an ophthalmic solution composition. The ophthalmic solution composition had a pH level of 6.73 and an osmotic pressure ratio of 1.02.

### Comparative Example 7

0.005 g of latanoprost and 0.25 g of polysorbate 80 that had been mixed previously were added to and dissolved into a small amount of purified water that had been heated to about 80°C. After returning the solution to a room temperature, 0.8 g of trometamol was added and dissolved into the solution, and a pH level was adjusted to about 6. 7 by using diluted hydrochloric acid or sodium hydroxide. 0. 51 g of sodium chloride was added to and dissolved into the solution, and purified water was added to adjust a total amount to 100 mL, thereby obtaining an ophthalmic solution composition. The ophthalmic solution composition had a pH level of 6. 68 and an osmotic pressure ratio of 1.01.

### Comparative Example 8

0.005 g of latanoprost was added to and dissolved into a small amount of purified water that had been heated to about 80°C. After returning the solution to a room temperature, 0.8 g of trometamol and 0.1 g of citric acid hydrate were added and dissolved into the solution, and a pH level was adjusted to about 6.7 by using diluted hydrochloric acid or sodium hydroxide. 0.55 g of sodium chloride was added to and dissolved into the solution, and purified water was added to adjust a total amount to 100 mL, thereby obtaining an ophthalmic solution composition. The ophthalmic solution composition had a pH level of 6.85 and an osmotic pressure ratio of 0.99.

### Comparative Example 9

0.005 g of latanoprost and 0.25 g of polysorbate 80 that had been mixed previously were added to and dissolved into a small amount of purified water that had been heated to about 80°C. After returning the solution to a room temperature, 0.2 g of glucose was added and dissolved into the solution, and a pH level was adjusted to about 6.7 by using diluted hydrochloric acid or sodium hydroxide. 0. 86 g of sodium chloride was added to and dissolved into the solution, and purified water was added to adjust a total amount to 100 mL, thereby obtaining an ophthalmic solution composition. The ophthalmic solution composition had a pH level of 6. 53 and an osmotic pressure ratio of 1.00.

### Comparative Example 10

0.005 g of latanoprost and 0.25 g of polysorbate 80 that had been mixed previously were added to and dissolved into a small amount of purified water that had been heated to about 80°C. After returning the solution to a room temperature, 4.0 g of mannitol was added and dissolved into the solution, and a pH level was adjusted to about 6.7 by using diluted hydrochloric acid or sodium hydroxide. 0. 17 g of sodium chloride was added to and dissolved into the solution, and purified water was added to adjust a total amount to 100 mL, thereby obtaining an ophthalmic solution composition. The ophthalmic solution composition had a pH level of 6. 56 and an osmotic pressure ratio of 1.00.

### Comparative Example 11

0.005 g of latanoprost and 0.25 g of polysorbate 80 that had been mixed previously were added to and dissolved into a small amount of purified water that had been heated to about 80°C. After returning the solution to a room temperature, 0.8 g of sorbitol was added and dissolved into the solution, and a pH level was adjusted to about 6.7 by using diluted hydrochloric acid or sodium hydroxide. 0. 75 g of sodium chloride was added to and dissolved into the solution, and purified water was added to adjust a total amount to 100 mL, thereby obtaining an ophthalmic solution composition. The ophthalmic solution composition had a pH level of 6.46 and an osmotic pressure ratio of 0.99.

### Comparative Example 12

0.005 g of latanoprost and 0.25 g of polysorbate 80 that had been mixed previously were added to and dissolved into a small amount of purified water that had been heated to about 80°C. After returning the solution to a room temperature, 0.1 g of dextran 70 was added and dissolved into the solution, and a pH level was adjusted to about 6.7 by using diluted hydrochloric acid or sodium-hydroxide. 0. 9 g of sodium chloride was added to and dissolved into the solution, and purified water was added to adjust a total amount to 100 mL, thereby obtaining an ophthalmic solution composition. The ophthalmic solution composition had a pH level of 6. 53 and an osmotic pressure ratio of 1.00.

### Comparative Example 13

0.005 g of latanoprost was added to and dissolved into a small amount of purified water that had been heated to about 80°C. After returning the solution to a room temperature, 0.8 g of trometamol was added and dissolved into the solution, and a pH level was adjusted to about 6. 7 by using diluted hydrochloric acid or sodium hydroxide. 0.52 g of sodium chloride was added to and dissolved into the solution, and an appropriate amount of purified water was added to adjust a total amount to 100 mL, thereby obtaining an ophthalmic solution composition. The ophthalmic solution composition had a pH level of 6.68 and an osmotic pressure ratio of 1.01.

### Comparative Example 14

0.005 g of latanoprost and 0.25 g of polysorbate 80 that had been mixed previously were added to and dissolved into a small amount of purified water that had been heated to about 80°C. After returning the solution to a room temperature, a pH level was adjusted to about 6. 7 by using diluted hydrochloric acid or sodium hydroxide. 0. 90 g of sodium chloride was added to and dissolved into the solution, and purified water was added to adjust a total amount to 100 mL, thereby obtaining an ophthalmic solution composition. The ophthalmic solution composition had a pH level of 5. 55 and an osmotic pressure ratio of 1.01.

### Comparative Example 15

0.005 g of latanoprost was added to and dissolved into a small amount of purified water that had been heated to about 80°C. After returning the solution to a room temperature, 0.1 g of citric acid hydrate was added and dissolved into the solution, and a pH level was adjusted to about 6.7 by using diluted hydrochloric acid or sodium hydroxide. 0.85 g of sodium chloride was added to and dissolved into the solution, and purified water was added to adjust a total amount to 100 mL, thereby obtaining an ophthalmic solution composition. The ophthalmic solution composition had a pH level of 6.71 and an osmotic pressure ratio of 1.00.

### Comparative Example 16

0.005 g of latanoprost was added to and dissolved into a small amount of purified water that had been heated to about 80°C. After returning the solution to a room temperature, 0.1 g of sodium edetate hydrate was added and dissolved into the solution, and a pH level was adjusted to about 6.7 by using diluted hydrochloric acid or sodium hydroxide. 0.87 g of sodium chloride was added to and dissolved into the solution, and purified water was added to adjust a total amount to 100 mL, thereby obtaining an ophthalmic solution composition. The ophthalmic solution composition had a pH level of 6.74 and an osmotic pressure ratio of 1.00.

### Comparative Example 17

0. 005 g of latanoprost was added to and dissolved into a small amount of purified water that had been heated to about 80°C. After returning the solution to a room temperature, 0.2 g of glucose was added and dissolved into the solution, and a pH level was adjusted to about 6.7 by using diluted hydrochloric acid or sodium hydroxide. 0. 86 g of sodium chloride was added to and dissolved into the solution, and purified water was added to adjust a total amount to 100 mL, thereby obtaining an ophthalmic solution composition. The ophthalmic solution composition had a pH level of 6.56 and an osmotic pressure ratio of 1.01.

### Comparative Example 18

0.005 g of latanoprost was added to and dissolved into a small amount of purified water that had been heated to about 80°C. After returning the solution to a room temperature, 4.0 g of mannitol was added and dissolved into the solution, and a pH level was adjusted to about 6. 7 by using diluted hydrochloric acid or sodium hydroxide. 0. 17 g of sodium chloride was added to and dissolved into the solution, and purified water was added to adjust a total amount to 100 mL, thereby obtaining an ophthalmic solution composition. The ophthalmic solution composition had a pH level of 6. 66 and an osmotic pressure ratio of 1.01.

### Comparative Example 19

0.005 g of latanoprost was added to and dissolved into a small amount of purified water that had been heated to about 80°C. After returning the solution to a room temperature, 0.8 g of sorbitol was added and dissolved into the solution, and a pH level was adjusted to about 6. 7 by using diluted hydrochloric acid or sodium hydroxide. 0. 75 g of sodium chloride was added to and dissolved into the solution, and purified water was added to adjust a total amount to 100 mL, thereby obtaining an ophthalmic solution composition. The ophthalmic solution composition had a pH level of 6.47 and an osmotic pressure ratio of 1.00.

### Comparative Example 20

0.005 g of latanoprost was added to and dissolved into a small amount of purified water that had been heated to about 80°C. After returning the solution to a room temperature, 0.1 g of dextran 70 was added and dissolved into the solution, and a pH level was adjusted to about 6. 7 by using diluted hydrochloric acid or sodium hydroxide. 0. 9 g of sodium chloride was added to and dissolved into the solution, and purified water was added to adjust a total amount to 100 mL, thereby obtaining an ophthalmic solution composition. The ophthalmic solution composition had a pH level of 6.24 and an osmotic pressure ratio of 1.00.

### Comparative Example 21

0.005 g of latanoprost and 0.25 g of polysorbate 80 that had been mixed previously were added to and dissolved into a small amount of purified water that had been heated to about 80°C. After returning the solution to a room temperature, 0.8 g of trometamol and 0.1 g of acetic acid were added to and dissolved into the solution, and a pH level was adjusted to about 6. 7 by using diluted hydrochloric acid or sodium hydroxide. 0.51 g of sodium chloride was added to and dissolved into the solution, and purified water was added to adjust a total amount to 100 mL, thereby obtaining an ophthalmic solution composition. The ophthalmic solution composition had a pH level of 6.76 and an osmotic pressure ratio of 1.01.

### Comparative Example 22

0.005 g of latanoprost was added to and dissolved into a small amount of purified water that had been heated to about 80°C. After returning the solution to a room temperature, 1.35 g of sodium hydrogenphosphate hydrate and 0.63 g of sodium dihydrogenphosphate dihydrate were added and dissolved into the solution, and a pH level was adjusted to about 6.7 by using diluted hydrochloric acid or sodium hydroxide. 0.40 g of sodium chloride was added to and dissolved into the solution, and purified water was added to adjust a total amount to 100 mL, thereby obtaining an ophthalmic solution composition. The ophthalmic solution composition had a pH level of 6.78 and an osmotic pressure ratio of 0.99.

### Test Example 3 (Reference)

### Storage Test

Each of the ophthalmic solution compositions obtained by Reference Examples 5 to 13 and Comparative Examples 7 to 22 and a commercially available product (each 2.5 mL) was packed in glass ampoules, and the ampoules were sealed by welding, followed by storage for 4 days, 8 days, and 28 days at 80°C. A latanoprost content after termination of each of the storage periods was measured by using high speed liquid chromatography. From a difference between each of the latanoprost contents after the storage and a content at the start of the test, a latanoprost remaining ratio was calculated. Results are shown in Table 3. It is considered that the glass ampoule substantially does not absorb latanoprost on its surface.

**[Table 3]**

| Storage Period | Latanoprost Remaining ratio (%) | | | |
|---|---|---|---|---|
| Sample | Start of test | After 4 days of storage | After 8 days of storage | After 28 days of storage |
| Example 5 Reference | 100.0 | 98.2 | 95.7 | 94.6 |
| Example 6 Reference | 100.0 | 98.1 | 96.9 | 93.3 |
| Example 7 Reference | 100.0 | 98.4 | 97.2 | 94.6 |
| Example 8 Reference | 100.0 | 98.7 | 95.9 | 89.9 |
| Example 9 Reference | 100.0 | 98.2 | 95.0 | 85.1 |
| Example 10 Reference | 100.0 | 98.0 | 95.3 | 83.5 |
| Example 11 Reference | 100.0 | 98.4 | 95.1 | 79.6 |
| Example 12 Reference | 100.0 | 96.2 | 94.6 | 91.4 |
| Example 13 Reference | 100.0 | 98.0 | 96.6 | 82.8 |
| Comp. Ex. 7 | 100.0 | 95.0 | 66.4 | 0.0 |
| Comp. Ex. 8 | 100.0 | 93.9 | 92.9 | 87.0 |
| Comp. Ex. 9 | 100.0 | 79.1 | 7.0 | 0.0 |
| Comp. Ex. 10 | 100.0 | 87.1 | 8.2 | 0.0 |
| Comp. Ex. 11 | 100.0 | 91.8 | 14.4 | 0.0 |
| Comp. Ex. 12 | 100.0 | 70.1 | 2.8 | 0.0 |
| Comp. Ex. 13 | 100.0 | 94.0 | 91.8 | 87.9 |
| Comp. Ex. 14 | 100.0 | 86.9 | 26.6 | 0.0 |
| Comp. Ex. 15 | 100.0 | 88.1 | 82.0 | 38.3 |
| Comp. Ex.16 | 100.0 | 92.8 | 84.7 | 54.2 |
| Comp. Ex. 17 | 100.0 | 95.8 | 93.8 | 68.5 |
| Comp. Ex. 18 | 100.0 | 97.6 | 94.4 | 83.8 |
| Comp. Ex. 19 | 100.0 | 95.6 | 91.6 | 77.9 |
| Comp. Ex. 20 | 100.0 | 91.6 | 88.8 | 60.4 |
| Comp. Ex. 21 | 100.0 | 92.2 | 54.7 | 4.6 |
| Comp. Ex. 22 | 100.0 | 92.1 | 87.0 | 64.7 |
| Commercially Available Product * | 100.0 | 84.5 | 76.6 | 33.0 |

| | | | | |
|---|---|---|---|---|
| *Xalatan ophthalmic solution (Lot No. 07AH007) | | | | |

### Test Example 4 (Reference)

### Adsorption Ratio Measurement Test

Adsorption of latanoprost contained in each of the ophthalmic solution compositions obtained by Reference Examples 5 to 13 and Comparative Examples 7 to 22 and a commercially available product was tested. After filling each of a polyethylene container and a glass ampoule with 2.5 mL of each of the samples, the polyethylene container was provided with an inner cap and an outer cap made from plastic, and the glass ampoule was sealed by welding, thereby giving eye drop preparations.

The eye drop preparations were stored for 7 days under the conditions of upright, still standing and at 60°C, and a latanoprost content at termination of the storage period was measured by using high speed liquid chromatography. Results are shown in Table 4. The content is expressed by way of a ratio (remaining ratio) to the content at the start of the test. The data of the polyethylene container was corrected by using a moisture evaporation value of a control wherein purified water was stored under the same conditions.

**[Table 4]**

| Storage Period | Latanoprost Remaining ratio (%) | | | Latanoprost |
|---|---|---|---|---|
| Sample | Start of test | (1) After 7 days of storage in glass container | (2) After 7 days of storage in polyethylene container | Adsorption Ratio (%) [(1)-(2)] |
| Example 5 Reference | 100.0 | 98.9 | 97.6 | 1.3 |
| Example 6 Reference | 100.0 | 98.9 | 97.8 | 1.1 |
| Example 7 Reference | 100.0 | 98.7 | 97.0 | 1.7 |
| Example 8 Reference | 100.0 | 99.3 | 96.8 | 2.5 |
| Example 9 Reference | 100.0 | 99.2 | 96.3 | 2.9 |
| Example 10 Reference | 100.0 | 99.0 | 95.7 | 3.3 |
| Example 11 Reference | 100.0 | 98.8 | 96.6 | 2.2 |
| Example 12 Reference | 100.0 | 98.9 | 97.0 | 1.9 |
| Example 13 Reference | 100.0 | 98.2 | 96.2 | 2.0 |
| Comp. Ex. 7 | 100.0 | 98.2 | 96.9 | 1.3 |
| Comp. Ex. 8 | 100.0 | 94.4 | 79.6 | 14.8 |
| Comp. Ex. 9 | 100.0 | 96.7 | 100.8 | -4.1 |
| Comp. Ex. 10 | 100.0 | 98.4 | 97.8 | 0.6 |
| Comp. Ex. 11 | 100.0 | 98.2 | 95.7 | 2.5 |
| Comp. Ex. 12 | 100.0 | 98.3 | 97.2 | 1.1 |
| Comp. Ex. 13 | 100.0 | 95.2 | 81.7 | 13.5 |
| Comp. Ex. 14 | 100.0 | 96.3 | 97.0 | -0.7 |
| Comp. Ex. 15 | 100.0 | 91.4 | 80.1 | 11.3 |
| Comp. Ex. 16 | 100.0 | 94.1 | 80.7 | 13.4 |
| Comp. Ex.17 | 100.0 | 98.7 | 85.8 | 12.9 |
| Comp. Ex. 18 | 100.0 | 100.2 | 85.0 | 15.2 |
| Comp. Ex. 19 | 100.0 | 99.3 | 85.0 | 14.3 |
| Comp. Ex. 20 | 100.0 | 97.6 | 85.9 | 11.7 |
| Comp. Ex. 21 | 100.0 | 97.7 | 97.2 | 0.5 |
| Comp. Ex. 22 | 100.0 | 94.7 | 81.9 | 12.8 |
| Commercially Available Product * | 100.0 | 91.2 | 79.3 | 11.9 |

| | | | | |
|---|---|---|---|---|
| * Xalatan ophthalmic solution (Lot No. 07AH007) | | | | |

From the above results, it was revealed that the formulations of Reference Examples 5 to 13 are excellent in both of adsorption suppression and degradation suppression since the formulations of Reference Examples 5 to 13 have the higher latanoprost remaining ratio itself as compared to the formulations of Comparative Examples 7 to 22 and the commercially available product and since the latanoprost adsorption ratio in the plastic container is not largely different from that of the glass container. For instance, though Comparative Examples 8, 13, 18, and 19 achieve the remaining ratios that are close to those of Examples, the adsorption ratios onto plastic containers of Comparative Examples 8, 13, 18, and 19 exceed 10% which are considerably larger than those of Examples, resulting in comprehensive judgment of considerable inferiority to Examples.

From the above results, it was revealed that the ophthalmic solution composition of this invention in a state of being packed in a plastic container is free from a problematic reduction in content of the active ingredient in the latanoprost-containing eye drop preparation and can be stably stored.

### Example 14 (Reference)

0.005 g of latanoprost and 0.25 g of polysorbate 80 that had been mixed previously were added to and dissolved into a small amount of purified water that had been heated to about 80°C. After returning the solution to a room temperature, 0.8 g of trometamol and 1.0 g of boric acid were added to and dissolved into the solution, and a pH level was adjusted to about 6.7 by using diluted hydrochloric acid or sodium hydroxide. Purified water was added to adjust a total amount to 100 mL, thereby obtaining an ophthalmic solution composition. The ophthalmic solution composition had a pH level of 6.77.

### Example 15 (Reference)

0.005 g of latanoprost and 0.25 g of polysorbate 80 that had been mixed previously were added to and dissolved into a small amount of purified water that had been heated to about 80°C. After returning the solution to a room temperature, 0.8 g of trometamol and 0.5 g of disodium hydrogenphosphate·12 hydrate were added to and dissolved into the solution, and a pH level was adjusted to about 6.7 by using diluted hydrochloric acid or sodium hydroxide. Purified water was added to adjust a total amount to 100 mL, thereby obtaining an ophthalmic solution composition. The ophthalmic solution composition had a pH level of 6.72.

### Comparative Example 23

0.005 g of latanoprost and 0.25 g of polysorbate 80 that had been mixed previously were added to and dissolved into a small amount of purified water that had been heated to about 80°C. After returning the solution to a room temperature, 0.8 g of trometamol was added to and dissolved into the solution, and a pH level was adjusted to about 6. 7 by using diluted hydrochloric acid or sodium hydroxide. 0.51 g of sodium chloride was added to and dissolved into the solution, and purified water was added to adjust a total amount to 100 mL, thereby obtaining an ophthalmic solution composition. The ophthalmic solution composition had a pH level of 6.63.

### Comparative Example 24

0.005 g of latanoprost and 0.25 g of polysorbate 80 that had been mixed previously were added to and dissolved into a small amount of purified water that had been heated to about 80°C. After returning the solution to a room temperature, 1.0 g of boric acid was added to and dissolved into the solution, and a pH level was adjusted to about 6. 7 by using hydrochloric acid or sodium hydroxide. Purified water was added to adjust a total amount to 100 mL, thereby obtaining an ophthalmic solution composition. The ophthalmic solution composition had a pH level of 6.82.

### Comparative Example 25

0.005 g of latanoprost and 0.25 g of polysorbate 80 that had been mixed previously were added to and dissolved into a small amount of purified water that had been heated to about 80°C. After returning the solution to a room temperature, 0.5 g of disodium hydrogenphosphate·12 hydrate was added to and dissolved into the solution, and a pH level was adjusted to about 6.7 by using hydrochloric acid or sodium hydroxide. Purified water was added to adjust a total amount to 100 mL, thereby obtaining an ophthalmic solution composition. The ophthalmic solution composition had a pH level of 6.72.

### Comparative Example 26

0.005 g of latanoprost and 0.25 g of polysorbate 80 that had been mixed previously were added to and dissolved into a small amount of purified water that had been heated to about 80°C. After returning the solution to a room temperature, a pH level was adjusted to about 6.7 by using diluted hydrochloric acid or sodium hydroxide. 0. 90 g of sodium chloride was added to and dissolved into the solution, and purified water was added to adjust a total amount to 100 mL, thereby obtaining an ophthalmic solution composition. The ophthalmic solution composition had a pH level of 5.55.

### Comparative Example 27

0. 005 g of latanoprost was added to and dissolved into a small amount of purified water that had been heated to about 80°C. After returning the solution to a room temperature, 0.8 g of trometamol was added to and dissolved into the solution, and a pH level was adjusted to about 6.7 by using diluted hydrochloric acid or sodium hydroxide. 0.52 g of sodium chloride was added to and dissolved into the solution, and purified water was added to adjust a total amount to 100 mL, thereby obtaining an ophthalmic solution composition. The ophthalmic solution composition had a pH level of 6.68.

### Test Example 5 (Reference)

### Storage Test (Glass Container)

Each of the ophthalmic solution compositions obtained by Reference Examples 14 and 15 and Comparative Examples 23 to 27 and a commercially available product (each 2.5 mL) was packed in glass ampoules, and the ampoules were sealed by welding, followed by storage for 4 days and 8 days at 80°C. A latanoprost content at the start of the test and after termination of each of the storage periods was measured by using high speed liquid chromatography, and a latanoprost remaining ratio in each of the storage periods was calculated. Results are shown in Table 5. It is considered that the glass ampoule substantially does not absorb latanoprost on its surface.

**[Table 5]**

| Storage Period | Latanoprost Remaining ratio (%) | | |
|---|---|---|---|
| Sample | Start of test | After 4 days of storage | After 8 days of storage |
| Example 14 Reference | 100.0 | 98.5 | 94.6 |
| Example 15 Reference | 100.0 | 97.2 | 94.8 |
| Comp. Ex. 23 | 100.0 | 95.0 | 66.4 |
| Comp. Ex. 24 | 100.0 | 90.6 | 12.4 |
| Comp. Ex. 25 | 100.0 | 19.9 | 0.0 |
| Comp. Ex. 26 | 100.0 | 86.9 | 26.6 |
| Comp. Ex. 27 | 100.0 | 94.0 | 91.8 |
| Xalatan Ophthalmic Solution | 100.0 | 84.5 | 76.6 |

| | | | |
|---|---|---|---|
| * Xalatan ophthalmic solution (Lot No. 07AH007) Reference | | | |

### Test Example 6 (Reference)

### Storage Test (Plastic Container)

Each of the ophthalmic solution compositions obtained by (Reference) Examples 14 and 15 and Comparative Examples 23 to 27 and a commercially available product (each 2.5 mL) was packed in polyethylene containers, and each of the containers were provided with an inner cap and an outer cap made from plastic, followed by storage for 7 days and 14 days under the conditions of upright still standing and 60°C. A latanoprost content at the start of the test and after termination of each of the storage periods was measured by using high speed liquid chromatography, and a latanoprost remaining ratio in each of the storage periods was calculated. Results are shown in Table 6. The data of the polyethylene container was corrected by using a moisture evaporation value of a control wherein purified water was stored under the same conditions.

**[Table 6]**

| Storage Period | Latanoprost Remaining ratio (%) | | |
|---|---|---|---|
| Sample | Start of test | After 7 days of storage | After 14 days of storage |
| Example 14 Reference | 100.0 | 94.6 | 94.1 |
| Example 15 Reference | 100.0 | 94.0 | 93.7 |
| Comp. Ex. 23 | 100.0 | 96.9 | 92.4 |
| Comp. Ex. 24 | 100.0 | 92.5 | 88.4 |
| Comp. Ex. 25 | 100.0 | 94.0 | 89.2 |
| Comp. Ex. 26 | 100.0 | 97.0 | 92.7 |
| Comp. Ex. 27 | 100.0 | 81.7 | 79.1 |
| Xalatan Ophthalmic Solution | 100.0 | 79.3 | 71.0 |

| | | | |
|---|---|---|---|
| * Xalatan ophthalmic solution (Lot No. 07AH007) #Reference | | | |

From the above results, it was revealed that the formulations of Reference Examples 14 and 15 are excellent in both of a suppression effect on latanoprost adsorption onto an inner surface of a plastic container and suppression effect on latanoprost heat degradation as compared to the formulations of Comparative Examples 23 to 27 and the commercially available product.

From the above results, it was revealed that a problematic reduction in content of the active ingredient does not occur in the composition for latanoprost-containing eye drop preparation and that stable storage is enabled in a state of being packed in a plastic container.

### Example 16

### Preparation of Eye Drop Preparation

In accordance with composition tables shown in Tables 8 to 12 (unit of content of each of components is weight/volume %; (w/v) %, unless otherwise noted), latanoprost, polysorbate 80, and glycerin that had been previously mixed were added to and dissolved into purified water of about 80°C, and hypromellose (hydroxypropylmethylcellulose 2910; dynamic viscosity: 6mm²/s) was added, followed by cooling to a room temperature. After that, disodium hydrogenphosphate 12hydrate, sodium dihydrogenphosphate dihydrate, trometamol, citric acid hydrate, D-mannitol, sodium chloride, and benzetonium chloride were added and dissolved into the solution, and pH was adjusted with hydrochloric acid or sodium hydroxide. Purified water was further added to adjust a total amount to 100 mL, thereby obtaining an ophthalmic solution (note that any component other than those described in the composition tables is not added in each of ophthalmic solutions).

2.5 mL of each of the ophthalmic solutions prepared as described above was packed in a polypropylene container or a polyethylene container each having a diameter of about 1.5 cm and a capacity of about 5 mL (without shrinkable film), and the container was stopped up with an inner spigot normally fitted with the container and a cap to give an eye drop preparation.

### [Stability Measurement Method]

2.5 mL of each of the ophthalmic solutions prepared as described above was packed in a polypropylene container or a polyethylene container each having a diameter of about 1.5 cm and a capacity of about 5 mL (without shrinkable film), and the container was stopped up with an inner spigot normally fitted with the container and a cap to give an eye drop preparation. The eye drop preparation was stored under the conditions of 40°C, relative humidity of 75%, shading, and upright still standing. After 30 days had passed, a latanoprost content was measured by high speed liquid chromatography, and a ratio of a latanoprost concentration after the storage to a latanoprost concentration before the start of the storage was used as a latanoprost remaining ratio.

Measurement conditions for the high speed liquid chromatography are as shown in Table 7.
The same measurement was conducted as comparative examples on Commercially Available Eye Drop Preparation 1 obtained by transferring a content of a commercially available latanoprost-containing eye drop preparation (trade name: Xalatan ophthalmic solution) into a polypropylene container same as that used for Eye Drop Preparations 1 to 15 and Commercially Available Eye Drop Preparation 2 which is the commercially available latanoprost-containing eye drop preparation (trade name: Xalatan ophthalmic solution) as it is.

**[Table 7]**

| Detector | Ultraviolet Absorptiometer (Measurement Wavelength: 210 nm) |
|---|---|
| Column | A stainless steel tube having an inner diameter of 4.6 mm and a length of 25 cm was filled with an octadecyl silylated silica gel for liquid chromatography obtained by chemical modification with fluorine-containing silicon. |
| Column Temperature | Constant temperature around 40°C |
| Mobile Phase | Methanol/water/phosphoric acid (1500:1000:1) |
| Flow Rate | Adjusted so that latanoprost retention time is about 11 minutes. |

**[Table 8]**

| | Eye Drop Preparation 1 | Eye Drop Reference Preparation 2 | Eye Drop Reference Preparation 3 | Eye Drop Reference Preparation 4 |
|---|---|---|---|---|
| Latanoprost | 0.005% | 0.005% | 0.005% | 0.005% |
| Disodium hydrogenphosphate 12hydrate | - | 0.5% | 0.5% | 0.5% |
| Sodium dihydrogenphosphate dihydrate | - | - | 0.25% | - |
| Trometamol | 0.8% | - | - | - |
| Citric acid hydrate | 0.1% | 0.1% | - | 0.1% |
| Glycerin | 1.0% | 1.0% | 1.0% | |
| D-mannitol | 1.0% | 1.0% | 1.0% | 1.0% |
| Polysorbate 80 | 0.05% | 0.05% | 0.05% | 0.05% |
| Hypromellose | 0.3% | 0.3% | 0.3% | 0.3% |
| Benzetonium chloride | 0.01% | 0.01% | 0.01% | 0.01% |
| Sodium chloride | - | 0.4% | 0.34% | 0.7% |
| Hydrochloric acid or sodium hydroxide | As required | As required | As required | As required |
| Purified water | As required | As required | As required | As required |
| pH | 6.71 | 6.68 | 6.70 | 6.66 |
| Osmotic pressure (mOsm) | 267 | 326 | 328 | 319 |
| Storage container material | PP | PP | PP | PP |
| Remaining ratio (%) | 100.1 | 99.3 | 99.5 | 97.9 |

| | | | | |
|---|---|---|---|---|
| Abbreviation for storage container material PP = polypropylene #Reference | | | | |

**[Table 9]**

| | Eye Drop Reference Preparation 5 | Eye Drop Reference Preparation 6 | Eye Drop Reference Preparation 7 | Eye Drop Reference Preparation 8 |
|---|---|---|---|---|
| Latanoprost | 0.005% | 0.005% | 0.005% | 0.005% |
| Disodium hydrogenphosphate 12hydrate | 0.5% | - | - | - |
| Sodium dihydrogenphosphate dihydrate | - | 0.25% | 0.25% | 0.25% |
| Trometamol | - | 0.8% | 0.8% | 0.8% |
| Citric acid hydrate | 0.1% | - | - | - |
| Glycerin | 1.0% | 1.0% | - | 1.0% |
| D-mannitol | 1.0% | 1.0% | 1.0% | - |
| Polysorbate 80 | 0.05% | 0.05% | 0.05% | 0.05% |
| Hypromellose | - | 0.3% | 0.3% | 0.3% |
| Benzetonium chloride | 0.01% | 0.01% | 0.01% | 0.01% |
| Sodium chloride | 0.4% | - | - | - |
| Hydrochloric acid or sodium hydroxide | As required | As required | As required | As required |
| Purified water | As required | As required | As required | As required |
| pH | 6.75 | 6.87 | 6.80 | 6.78 |
| Osmotic pressure (mOsm) | 325 | 295 | 194 | 232 |
| Storage container material | PP | PP | PP | PP |
| Remaining ratio (%) | 100.8 | 99.7 | 99.1 | 101.1 |

| | | | | |
|---|---|---|---|---|
| Abbreviation for storage container material: PP = polypropylene #*Reference* | | | | |

**[Table 10]**

| | Eye Drop Reference Preparation 9 | Eye Drop Reference Preparation 10 | Eye Drop Reference Preparation 11 | Eye Drop Reference Preparation 12 |
|---|---|---|---|---|
| Latanoprost | 0.005% | 0.005% | 0.005% | 0.005% |
| Disodium hydrogenphosphate 12hydrate | - | - | - | - |
| Sodium dihydrogenphosphate dihydrate | 0.25% | - | - | - |
| Trometamol | 0.8% | 0.8% | 0.8% | 0.8% |
| Citric acid hydrate | - | 0.1% | 0.1% | 0.1% |
| Glycerin | 1.0% | - | 1.0% | 1.0% |
| D-mannitol | 1.0% | 1.0% | - | - |
| Polysorbate 80 | 0.05% | 0.05% | 0.05% | 0.05% |
| Hypromellose | - | - | 0.3% | - |
| Benzetonium chloride | 0.01% | 0.01% | 0.01% | 0.01% |
| Sodium chloride | - | 0.3% | 0.18% | 0.18% |
| Hydrochloric acid or sodium hydroxide | As required | As required | As required | As required |
| Purified water | As required | As required | As required | As required |
| pH | 6.70 | 6.93 | 6.90 | 6.95 |
| Osmotic pressure (mOsm) | 292 | 266 | 267 | 268 |
| Storage container material | PP | PP | PP | PP |
| Remaining ratio (%) | 99.8 | 98.9 | 98.2 | 98.4 |

| | | | | |
|---|---|---|---|---|
| Abbreviation for storage container material: PP = polypropylene #Reference | | | | |

**[Table 11]**

| | Eye Drop Reference Preparation 13 | Eye Drop Reference Preparation 14 | Eye Drop Reference Preparation 15 | Eye Drop Reference Preparation 16 |
|---|---|---|---|---|
| Latanoprost Disodium hydrogenphosphate | 0.005% | 0.005% | 0.005% | Preparation 16 0.005% |
| Disodium hydrogenphosphate 12hydrate Sodium dihydrogenphosphate | - | - | - | - |
| Sodium dihydrogenphosphate dihydrate | - | - | - | - |
| Trometamol | 0.8% | 0.8% | 0.8% | 0.8% |
| Citric acid hydrate | 0.1% | 0.1% | 0.1% | 0.1% |
| Glycerin | 1.0% | 1.0% | - | 1.1% |
| D-mannitol | - | - | 1.0% | 1.0% |
| Polysorbate 80 | 0.05% | 0.05% | 0.05% | 0.05% |
| Hypromellose | - | 0.3% | 0.3% | - |
| Benzetonium chloride | 0.01% | 0.01% | 0.01% | 0.005% |
| Sodium chloride hydrochloric acid or sodium | - | - | - | - |
| Hydrochloric acid or sodium hydroxide | As required | As required | As required | As required |
| Purified water | As required | As required | As required | As required |
| pH | 6.84 | 6.65 | 6.77 | 6.68 |
| Osmotic pressure (mOsm) | 209 | 207 | 169 | 274 |
| Storage container material | PP | PP | pp | PE |
| Remaining ratio (%) | 99.3 | 98.6 | 98.1 | 97.4 |

| | | | | |
|---|---|---|---|---|
| Abbreviation for storage container material: PP = polypropylene; PE = polyethylene #Reference | | | | |

**[Table 12]**

| | Commercially Available Eye Drop Preparation 1 (Xalatan ophthalmic solution) | Commercially Available Eye Drop Preparation 2 (Xalatan ophthalmic solution) |
|---|---|---|
| Latanoprost | 0.005% | 0.005% |
| Disodium hydrogenphosphate 12hydrate | Other detailed components and contents are unknown | Other detailed components and contents are unknown |
| Sodium dihydrogenphosphate dihydrate | | |
| Trometamol | | |
| Citric acid hydrate | | |
| Glycerin | | |
| D-mannitol | | |
| Polysorbate 80 | | |
| Hypromellose | | |
| Benzetonium chloride | | |
| Sodium chloride | | |
| Hydrochloric acid or sodium hydroxide | | |
| Purified water | | |
| pH | 6.80 | 6.80 |
| Osmotic pressure (mOsm) | 272 | 272 |
| Storage container material | PP | The commercially available product was used as it was |
| Remaining ratio (%) | 90.6 | 91.3 |

| | | |
|---|---|---|
| Abbreviation for storage container material: PP = polypropylene | | |

### [Results]

Shown in Tables 8 to 12 are results of the storage test. From the above results, it was revealed that the ophthalmic solution of this invention for preventing or treating glaucoma or ocular hypertension achieved the latanoprost remaining ratio of 97.0% or more after the 30 days of storage under the conditions of 40°C, relative humidity of 75%, shading, and upright still standing in the case where 2.5 mL of the ophthalmic solution was packed in the polypropylene or polyethylene container having the diameter of about 1.5 cm and the capacity of about 5 mL. Among others, Eye Drop Preparation 1, Eye Drop Preparation 2, Eye Drop Preparation 3, Eye Drop Preparation 5, Eye Drop Preparation 6, Eye Drop Preparation 7, Eye Drop Preparation 8, Eye Drop Preparation 9, Eye Drop Preparation 10, Eye Drop Preparation 11, Eye Drop Preparation 12, Eye Drop Preparation 13, Eye Drop Preparation 14, and Eye Drop Preparation 15 achieved the latanoprost remaining ratio of 98.0% or more, and, particularly, Eye Drop Preparation 1, Eye Drop Preparation 2, Eye Drop Preparation 3, Eye Drop Preparation 5, Eye Drop Preparation 6, Eye Drop Preparation 7, Eye Drop Preparation 8, Eye Drop Preparation 9, and Eye Drop Preparation 13 achieved the latanoprost remaining ratio of 99.0% or more.

### Example 17

### Preparation of Eye Drop Preparation

In accordance with a composition table shown in Tables 13 (unit of content of each of components is weight/volume %; (w/v)%, unless otherwise noted), latanoprost, polysorbate 80, and glycerin that had been previously mixed were added to and dissolved into purified water of about 80°C, and hypromellose (hydroxypropylmethylcellulose 2910; dynamic viscosity: 6 mm²/s) was added, followed by cooling to a room temperature. After that, trometamol, citric acid hydrate, D-mannitol, and benzalkonium chloride were added and dissolved into the solution, and pH was adjusted with diluted hydrochloric acid. Purified water was further added to adjust a total amount to 100 mL, thereby obtaining an ophthalmic solution.

**[Table 13]**

| | Eye Drop Preparation A | Eye Drop Preparation B | Eye Drop Preparation C | Eye Drop Preparation D |
|---|---|---|---|---|
| Latanoprost | 0.005% | 0.005% | 0.005% | 0.005% |
| Trometamol | 0.80% | 0.80% | 0.80% | 0.80% |
| Citric acid hydrate | 0.10% | 0.10% | 0.10% | 0.10% |
| Diluted hydrochloric acid | As required | As required | As required | As required |
| D-mannitol | 1.00% | 1.00% | 1.00% | 1.00% |
| Glycerin | 1.10% | 1.10% | 1.10% | 1.10% |
| Hypromellose | 0.30% | 0.30% | 0.30% | 0.30% |
| Polysorbate 80 | 0.05% | 0.05% | 0.05% | 0.05% |
| Benzalkonium chloride solution (10%) | 0.20% | 0.20% | 0.11% | 0.11% |
| Purified water | As required | As required | As required | As required |
| pH | 6.8 | 6.8 | 6.6 | 6.6 |
| Osmotic pressure ratio | 1.0 | 1.0 | 1.0 | 1.0 |
| 40°C 75% RH Remaining ratio of latanoprost after 2 months or 6 months of storage | 95.9% (6M) | 98.3% (6M) | 96.9% (2M) | 99.4% (2M) |

| | | | | |
|---|---|---|---|---|
| Container material: polyethylene | | | | |

2.5 mL of each of the ophthalmic solutions prepared as described above was packed in a polyethylene container having a diameter of about 1.5 cm and a capacity of about 5 mL (without shrinkable film), and the container was stopped up with an inner spigot normally fitted with the container and a cap to give an eye drop preparation.

### [Stability Measurement Method]

The eye drop preparation was stored under the conditions of 40°C, relative humidity of 75%, shading, and upright still standing. After 2 months or 6 months had passed, a latanoprost content was measured by high speed liquid chromatography, and a ratio of a latanoprost concentration after the storage to a latanoprost concentration before the start of the storage was used as a latanoprost remaining ratio. Measurement conditions for the high speed liquid chromatography are as shown in Table 14.

**[Table 14]**

| Detector | Ultraviolet Absorptiometer (Measurement Wavelength: 210 nm) |
|---|---|
| Column | A stainless steel tube having an inner diameter of 3.0 mm and a length of 25 cm was filled with 5 µm of an octadecyl silylated silica gel for liquid chromatography. |
| Column Temperature | Constant temperature around 40°C |
| Mobile Phase | 2.2 g of 1-sodium octanesulfonate was dissolved into 1000 mL of acetonitrile/water/phosphoric acid mixture (650:350:1) |
| Flow Rate | Adjusted so that latanoprost retention time is about 6 minutes. |

### [Results]

As shown in Table 13, it is apparent that each of the eye drop preparations has good stability. Particularly, Eye Drop Preparations A and B maintain the remaining ratios of 95% or more after 6 months of storage, and such results support the capability of storage at room temperature.

### Industrial Applicability

According to the ophthalmic solution of this invention, it is possible to provide an ophthalmologic agent for treating glaucoma that can be packed in a plastic container and stored at a room temperature.

Therefore, as compared to the conventional examples that require refrigerated storage, the ophthalmic solution is improved in usability and is used advantageously in the medical field.

## Claims

1. An ophthalmic composition for eye drop preparation, comprising
| | |
|---|---|
| 0.005 (w/v)% | of latanoprost, |
| 0.4 to 1.2 (w/v)% | of trometamol, |
| 0.05 to 0.15 (w/v)% | of citric acid hydrate, |
| 0.5 to 1.5 (w/v)% | of D-mannitol, |
| 0.55 to 1.65 (w/v)% | of glycerin, |
| 0.15 to 0.45 (w/v)% | of hypromellose, |
| 0.025 to 0.375 (w/v)% | of Polysorbate 80, and |
| 0.0055 to 0.030 (w/v)% | of benzalkonium chloride. |

2. The ophthalmic solution according to claim 1, which achieves a latanoprost remaining ratio in the ophthalmic solution after 30 days of storage under the conditions of 40°C, relative humidity of 75%, shading, and upright still standing of 97% or more, when packed in a polypropylene or polyethylene container not covered with the shrinkable film, and the container is stopped up with an inner spigot normally fitted with the container and a cap.

## Patentansprüche

1. Ophthalmische Zusammensetzung für eine Augentropfenformulierung, umfassend
| | |
|---|---|
| 0,005 (Gewicht/Volumen)% | Latanoprost, |
| 0,4 bis 1,2 (Gewicht/Volumen)% | Trometamol, |
| 0,05 bis 0,15 (Gewicht/Volumen)% | Zitronensäurehydrat, |
| 0,5 bis 1,5 (Gewicht/Volumen)% | D-Mannitol, |
| 0,55 bis 1,65 (Gewicht/Volumen)% | Glycerin, |
| 0,15 bis 0,45 (Gewicht/Volumen)% | Hypromellose, |
| 0,025 bis 0,375 (Gewicht/Volumen)% | Polysorbat 80 und |
| 0,0055 bis 0,030 (Gewicht/Volumen)% | Benzalkoniumchlorid. |

2. Die ophthalmische Lösung nach Anspruch 1, die ein Verhältnis an verbleibendem Lantanoprost in der opthalmischen Lösung nach 30 Tagen Lagerung von 97% oder mehr leistet, unter Bedingungen von 40°C bei einer relativen Luftfeuchigkeit 75%, Abschattung und aufrechtem Stehen, wenn diese in einem Polypropylen- oder Polyethylencontainer, der nicht mit einer Schrumpffolie bedeckt ist, verpackt ist, und wenn der Container mit einem Innenstutzen, der normalerweise den Container verschließt, und mit einer Kappe verschlossen ist.

## Revendications

1. Composition à usage ophtalmique pour préparation de gouttes oculaires, comprenant
| | |
|---|---|
| 0,005 % (p/v) | de latanoprost, |
| 0,4 à 1,2 % (p/v) | de trométamol, |
| 0,05 à 0,15 % (p/v) | d'acide citrique hydraté, |
| 0,5 à 1,5 % (p/v) | de D-mannitol, |
| 0,55 à 1,65 % (p/v) | de glycérine, |
| 0,15 à 0,45 % (p/v) | d'hypromellose, |
| 0,025 à 0,375 % (p/v) | de Polysorbate 80, et |
| 0,0055 à 0,030 % (p/v) | de chlorure de benzalkonium. |

2. Solution à usage ophtalmique selon la revendication 1, qui atteint un taux résiduel de latanoprost dans la solution à usage ophtalmique après 30 jours de stockage dans des conditions à 40°C sous une humidité relative de 75 %, à l'ombre et en position verticale, de 97 % ou plus, quand elle est conditionnée dans un récipient en polypropylène ou en polyéthylène non recouvert de film étirable, le récipient étant bouché par un robinet intérieur normalement ajusté au récipient et un capuchon.
